# FASCICULE DE BREVET EUROPEEN

(11) **EP 2 550 249 B1**
(45) Date de publication et mention de la délivrance du brevet: **07.10.2015**
(21) Numéro de dépôt: 11717325.2
(22) Date de dépôt: 21.03.2011
(51) Int. Cl.: C07C 67/08, C07C 67/58

(54) **PROCEDE D'OBTENTION D'UN ESTER D'ACIDE CARBOXYLIQUE**
VERFAHREN ZUR HERSTELLUNG EINES CARBONSÄUREESTERS
METHOD FOR PRODUCING A CARBOXYLIC ACID ESTER

(30) Priorité: 25.03.2010 FR 1052163
(43) Date de publication de la demande: 30.01.2013
(73) Titulaire: Rhodia Poliamida E Especialidades Ltda, Sao Paulo - SP (BR)
(72) Inventeur: MARENCO, Carlos, Eduardo, 13105-800 Campinas (SP) (BR); MARTINS, Sergio, 13083-000 Campinas (SP) (BR); BRESCIANI, Renato, 13083-130 Campinas (BR); SCHWARTZ, Joël, 69300 Caluire (FR)
(74) Mandataire: Ridray, Annabelle
(86) Numéro de dépôt international: PCT/IB2011/000596
(87) Numéro de publication internationale: WO 2011/117707

(56) Documents cités:
- WO-A1-01/46117
- WO-A1-98/42652
- GB-A- 934 027
- US-A- 2 001 926
- US-A- 2 147 341
- US-A- 2 787 636
- US-A1- 2009 005 588

## Description

La présente invention concerne un procédé d'obtention d'un ester d'acide carboxylique.

L'invention vise notamment l'obtention des esters de l'acide acétique et plus particulièrement l'acétate d'éthyle.

Les esters de l'acide acétique, en particulier l'acétate d'éthyle, sont utilisés généralement comme solvants organiques. En particulier, l'acétate d'éthyle est utilisé notamment dans le domaine de la cosmétique, de la parfumerie, dans les colles, peintures et vernis.

Selon l'application visée, une pureté plus ou moins élevée est demandée et il est courant de requérir que la quantité d'acide acétique présent dans l'acétate d'éthyle soit inférieure à 0,01 % en masse.

Ainsi, les procédés d'obtention de l'acétate d'éthyle doivent conduire à un produit de qualité. Etant donné que l'acétate d'éthyle est un produit de consommation courante, à gros volume, il importe que son procédé de d'obtention soit le plus performant possible, en termes de productivité et de bilan énergétique.

Les procédés classiques de fabrication de l'acétate d'éthyle prévoient une étape d'estérification couplée à une étape de distillation, comme décrit par exemple dans le brevet GB1173089 ou US2787636. Il peut également s'agir d'une distillation réactive telle que décrite dans la demande EP1220829, WO014846117 ou WO9842652.

Les étapes de réaction/distillation sont généralement suivies d'une étape de séparation des phases organique et aqueuse issues de la tête de distillation, comme décrit dans le brevet US4314947.

Pour répondre aux exigences de qualité de l'acétate d'éthyle, le brevet US1400849 décrit la purification ultérieure de l'acétate d'éthyle pour séparer le reste d'éthanol par distillation.

Toutefois, de tels procédés sont consommateurs d'énergie et il existe un besoin de réduire les coûts énergétiques de production dans ce domaine.

L'objectif de la présente invention est donc de fournir un procédé d'obtention d'un ester d'acide carboxylique qui soit amélioré en termes d'économie de procédé.

Dans ce but, la présente invention a pour objet un procédé d'obtention d'un ester d'acide carboxylique à partir d'une phase organique issue d'une réaction d'estérification entre un acide carboxylique et un alcool, ladite phase organique comprenant au moins majoritairement l'ester d'acide carboxylique et minoritairement l'alcool, de l'eau et des traces d'acide carboxylique, caractérisé par le fait qu'il comprend une étape de séparation au cours de laquelle la phase organique est appauvrie en alcool et débarrassée de l'acide carboxylique par une opération d'extraction liquide/liquide par mise en contact de ladite phase organique avec une phase aqueuse permettant d'extraire l'alcool et l'acide carboxylique présents dans la phase organique vers la phase aqueuse et par le fait, que la phase organique comprenant au moins majoritairement l'ester d'acide carboxylique et minontairement l'alcool, de l'eau et des traces d'acide carboxylique est issue des étapes suivantes :
a) réaction d'estérification entre l'acide carboxylique et l'alcool en présence d'un catalyseur homogène ou hétérogène,
b) distillation du milieu réactionnel issu de l'étape a), permettant d'obtenir en tête de colonne de distillation un flux vapeur comprenant majoritairement l'ester d'acide carboxylique et minoritairement de l'alcool, de l'eau et des traces d'acide carboxylique,
c) condensation du flux vapeur issue de l'étape b)
d) séparation des phases organique et aqueuse, notamment par décantation
et par le fait que l'alcool et l'acide carboxylique sont introduits à l'étape a) avec un ratio molaire acide carboxylique/alcool compris entre 7 et 25 ; l'acide carboxylique étant l'acide acétique et l'alcool étant choisi parmi l'éthanol, le butanol, le n-propanol et le cyclohexanol, et par le fait que le ratio entre les débits des phases organique et aqueuse au début de l'étape d'extraction liquide/liquide est compris entre 4 et 8

Dans le présent texte, on entend par « une phase comprenant majoritairement un composé donné », une phase dans laquelle le composé donné représente au moins à 85 % de sa masse, de préférence au moins 90 % de sa masse.

Par « une phase comprenant minoritairement un composé donné », on entend une phase dans laquelle le composé donné représente moins de 15 % de sa masse, de préférence moins de 10 % de sa masse.

Par « traces », on entend moins de 0,02 % en masse, notamment entre 0,01 et 0,02 % en masse du composé concerné.

Par « appauvrie en alcool », on entend qu'au moins 10 % en masse de l'alcool a été extrait de la phase organique vers la phase aqueuse, de préférence au moins 20 % en masse, et encore plus préférentiellement au moins 30 % en masse.

Par « débarrassée de l'acide carboxylique » ou « exempte », on entend que la phase organique comprend moins de 0,01 % en masse, notamment entre 0,001 et 0,01 % en masse d'acide carboxylique.

Au sens de la présente invention, les pourcentages en masse sont exprimés en pourcentage en masse par rapport à la masse totale de la phase (ou du flux) concernée.

Conformément au procédé selon l'invention, la phase organique est appauvrie en alcool et débarrassée de l'acide carboxylique par une opération d'extraction liquide/liquide.

L'extraction liquide/liquide consiste à séparer sélectivement un ou plusieurs composés d'un mélange sur la base de propriétés chimiques ou physiques. Il s'agit de l'extraction d'une substance qui est dissoute dans un solvant, à l'aide d'un autre solvant, appelé solvant d'extraction. Le principe physique est la différence de solubilité du produit à extraire entre les deux phases liquides.

Dans le cas de la présente invention, on extrait l'alcool présent dans la phase organique comprenant majoritairement l'ester de l'acide carboxylique et minoritairement l'alcool, de l'eau et des traces d'acide carboxylique avec une phase aqueuse ne contenant pas les composés à extraire et notamment l'alcool. De préférence, la phase aqueuse est constituée majoritairement d'eau, avantageusement il s'agit d'eau pure.

La phase aqueuse et la phase organique sont introduites de préférence à contre-courant par exemple dans un extracteur liquide/liquide fonctionnant en continu.

On obtient d'une part, une phase organique enrichie en ester d'acide carboxylique, appauvrie en alcool et débarrassée de l'acide carboxylique, et d'autre part, une phase aqueuse comprenant majoritairement de l'eau, de l'alcool et des traces d'acide carboxylique.

Selon un mode préféré de réalisation de l'invention, la phase organique enrichie en ester d'acide carboxylique, appauvrie en alcool et débarrassée de l'acide carboxylique a la composition suivante :
- de 85 à 98 % en masse, de préférence de 90 à 95 % en masse d'ester d'acide carboxylique,
- de 0,5 à 3 % en masse, de préférence de 1 à 2 % en masse d'alcool,
- de 2 à 8 % en masse, de préférence de 3 à 7 % en masse d'eau.

Selon un mode préféré de réalisation de l'invention, la phase aqueuse comprenant majoritairement de l'eau, de l'alcool et des traces d'acide carboxylique a la composition suivante :
- de 85 à 98 % en masse, de préférence de 90 à 95 % en masse d'eau,
- de 2 à 6 % en masse, de préférence de 3 à 5 % en masse d'alcool,
- de 2 à 8 % en masse, de préférence de 4 à 6 % en masse d'ester d'acide carboxylique,
- des traces d'acide carboxylique.

L'opération d'extraction liquide/liquide peut être conduite à une température comprise entre 15 et 50°C, de préférence entre 20 et 40°C.

Avantageusement, l'opération d'extraction liquide/liquide est mise en oeuvre à pression atmosphérique ou à une pression légèrement inférieure ou supérieure, par exemple à une pression comprise entre 0,5 et 5 bars absolus.

Selon l'invention, le ratio entre les débits des phases organique et aqueuse au début de l'étape d'extraction liquide/liquide est compris entre 4 et 8. Ce ratio est optimisé pour réduire la consommation globale d'énergie.

Le nombre d'étages théoriques de l'extracteur liquide/liquide est également optimisé de manière à réduire d'au moins 10 %, de préférence d'au moins 20 % et en particulier d'au moins 30 % la quantité d'alcool présente dans la phase organique qui est soumise à l'opération d'extraction liquide/liquide.

Selon le procédé de l'invention, l'étape d'extraction liquide/liquide permet généralement de purifier l'ester d'acide carboxylique d'au moins 1 %, de préférence de 1 à 2 % (absolu).

Selon l'invention, la phase organique comprenant majoritairement l'ester de l'acide carboxylique et minoritairement l'alcool, de l'eau et des traces d'acide carboxylique, provient d'un procédé d'estérification en présence d'un catalyseur homogène ou hétérogène suivie par une distillation.

Plus précisément, le procédé comprend les étapes suivantes :
a) réaction d'estérification entre l'acide carboxylique et l'alcool, en présence d'un catalyseur homogène ou hétérogène,
b) distillation du milieu réactionnel issu de l'étape a), permettant d'obtenir en tête de colonne de distillation un flux vapeur comprenant majoritairement l'ester d'acide carboxylique et minoritairement de l'alcool, de l'eau et des traces d'acide carboxylique,
c) condensation du flux vapeur issu de l'étape b),
d) séparation des phases organique et aqueuse, notamment par décantation.

Conformément à un mode de réalisation du procédé de l'invention, on effectue une réaction d'estérification de l'acide carboxylique a), par un alcool de préférence en présence d'un catalyseur acide.

L'acide carboxylique à estérifier est l'acide acétique.

L'acide carboxylique à estérifier est avantageusement introduit pur ou en solution aqueuse très concentrée. Le procédé de l'invention n'exclut pas la présence d'eau dans l'acide carboxylique. Cependant, il est préférable d'utiliser de l'acide carboxylique pur du fait de la nécessité ultérieure d'éliminer l'eau présente dans l'ester d'acide carboxylique obtenu en fin de procédé.

L'alcool mis en jeu est un alcool à chaîne alkyle linéaire ou ramifiée ayant de 1 à 6 atomes de carbone ou un alcool à chaîne cycloalkyle ayant 5 ou 6 atomes de carbone.

On préfère notamment les alcools à bas point d'ébullition, notamment inférieur à 170°C, de préférence inférieur à 165°C.

Ainsi, l'alcool à chaîne alkyle est choisi parmi l'éthanol, le butanol, le n-propanol, de préférence l'éthanol.

L'alcool à chaîne cycloalkyle est le cyclohexanol.

Selon l'invention, l'acide carboxylique est introduit en excès par rapport à l'alcool.

Un excès de l'un des réactifs peut être avantageux pour déplacer l'équilibre de la réaction vers la production d'ester d'acide carboxylique.

Ainsi le ratio molaire entre l'acide carboxylique et l'alcool est compris entre 7 et 25, notamment entre 9 et 20.

La borne supérieure, pour des raisons économiques, est avantageusement choisie inférieure à 25. Le ratio précisément défini correspond au ratio molaire des réactifs en début de réaction.

Le catalyseur intervenant dans le procédé de l'invention est un catalyseur acide protonique. Il peut s'agir d'un catalyseur homogène ou hétérogène.

Selon un premier mode, le catalyseur est un catalyseur acide hétérogène.

Les catalyseurs acides hétérogènes de l'invention sont préférentiellement des résines sulfoniques ou des zéolithes.

Les zéolithes qui peuvent être utilisées sont par exemple celles citées dans le document WO 2007/099071.

Les résines qui conviennent à la présente invention peuvent être constituées d'un squelette polystyrénique ou polyacrylique qui porte des groupes fonctionnels sulfoniques.

Ainsi, on peut mettre en oeuvre les résines acides sulfoniques SO₃H ou carboxyliques COOH existant sur le marché, résines commercialisées sous différentes dénominations commerciales.

On peut citer, entre autres, les résines d'estérification suivantes : Amberlyst® 15 de Rohm Haas, Amberlite® IR-120H de Rohm Haas, Lewatit® 2631 de Bayer et K1431 de Bayer.

L'acidité de ces résines est par exemple comprise entre 1 et 10 eq/kg (H+).

Ces résines sont notamment mises en oeuvre en lit fixe ou fluidisé, de préférence en lit fixe.

Selon un second mode, le catalyseur est un catalyseur acide homogène.

Par acide fort, on désigne dans la présente invention, un acide présentant un pKa dans l'eau inférieur à 2, de préférence inférieur à 1.

Le pKa est défini comme suit pKa = - log Ka, Ka étant la constante de dissociation ionique du couple acide/base à température ambiante (généralement 25°C), lorsque l'eau est utilisée comme solvant.

Parmi les acides répondant à cette définition, il est préférable d'utiliser un acide ne conduisant pas à des réactions parasites gênantes pour le procédé d'estérification et en particulier ne présentant pas de caractère oxydant, comme l'acide nitrique.

On peut citer comme acide fort homogène plus particulièrement l'acide sulfurique, les acides sulfoniques et leurs mélanges.

Comme acides sulfoniques, on peut mentionner notamment l'acide fluorosulfonique, l'acide chlorosulfonique ou l'acide trifluorométhanesulfonique, l'acide méthanesulfonique, l'acide éthanesulfonique, l'acide camphène-sulfonique, l'acide benzènesulfonique, les acides toluènesulfoniques, les acides xylènesulfoniques, les acides naphtalènesulfoniques.

Parmi ces acides, le catalyseur préféré est choisi parmi l'acide para-toluènesulfonique ou l'acide méthanesulfonique, de préférence l'acide méthanesulfonique.

La quantité de catalyseur introduite est de préférence comprise entre 0,1 et 2 % en masse par rapport au milieu réactionnel en début de réaction.

Il est possible d'introduire d'autres composés dans la réaction, par exemple des inhibiteurs de corrosion. Il peut s'agir notamment de sulfate de cuivre(II).

Conformément au procédé de l'invention, la réaction d'estérification peut être conduite selon un mode continu ou discontinu.

Selon un mode préféré de réalisation de l'invention, le procédé est un procédé en continu.

Dans le procédé de l'invention, l'acide carboxylique et l'alcool peuvent être introduits seuls ou en mélange, de préférence en mélange.

Selon un mode de réalisation de l'invention, on met en oeuvre l'étape a) d'estérification a lieu en présence d'un catalyseur acide à une température au moins égale à 50°C.

De façon avantageuse, la température de la réaction est comprise entre 50 et 150°C, de préférence entre 100 et 130°C.

La réaction est préférentiellement conduite à pression atmosphérique. Une pression légèrement supérieure ou inférieure à la pression atmosphérique peut également convenir. Ainsi, le procédé de l'invention peut être mise en oeuvre, par exemple, à une pression absolue comprise entre 0,5 et 5 bars absolus.

Selon un mode de réalisation de l'invention, le mélange réactionnel précédemment obtenu à l'étape a) est ensuite soumis à une opération de distillation b) afin d'obtenir en tête de distillation un flux vapeur comprenant majoritairement l'ester d'acide carboxylique et minoritairement l'alcool, de l'eau et des traces d'acide carboxylique.

L'opération de distillation b) est de préférence conduite dans une colonne de distillation. Le point d'alimentation où est introduit le mélange réactionnel est en général sensiblement à mi-hauteur de la colonne de distillation. Il peut être également situé plus bas et à une hauteur comprise entre la mi-hauteur et le pied de la colonne.

La température en pied de distillation est de préférence comprise entre 50 et 150°C, de préférence entre 100 et 130°C.

La pression définie en tête de distillation est de préférence comprise entre 0,5 et 5 bars absolus, avantageusement la pression en tête de distillation est comprise entre 1 et 2 bars absolus.

De façon avantageuse, l'opération de distillation b) permet l'obtention :
- d'un flux vapeur en tête de distillation comprenant majoritairement l'ester d'acide carboxylique, minoritairement de l'alcool et éventuellement de l'eau, et
- d'un milieu en pied de distillation comprenant majoritairement de l'acide carboxylique, minoritairement de l'alcool et de l'ester et éventuellement du catalyseur.

Dans le procédé de l'invention, le flux vapeur en tête de distillation comprend notamment :
- 75 à 98 % en masse d'ester de l'acide carboxylique,
- 0 à 17 % en masse d'eau,
- 0 à 8 % en masse d'alcool,
- des traces d'acide carboxylique,

Le flux vapeur en tête de distillation comprend encore plus préférentiellement :
- 85 à 95 % en masse d'ester de l'acide carboxylique,
- 0 à 10 % en masse d'eau,
- 0 à 5 % en masse d'alcool,
- de traces d'acide carboxylique.

Par « traces d'acide carboxylique », on entend moins de 0,02 % en masse de préférence entre 0,001 et 0,02 % en masse d'acide carboxylique.

Dans le procédé de l'invention, le milieu en pied de distillation comprend notamment :
- 60 à 98 % en masse d'acide carboxylique,
- 0 à 15 % en masse d'ester de l'acide carboxylique,
- 0 à 10 % en masse d'eau,
- 0 à 15 % en masse d'alcool.

Le milieu en pied de distillation comprend préférentiellement :
- 80 à 95 % en masse d'acide carboxylique,
- 2 à 15 % en masse d'ester de l'acide carboxylique,
- 2 à 8 % en masse d'eau,
- 0,1 à 2 % en masse d'alcool.

Le milieu en pied de distillation peut en outre contenir jusqu'à 2 % en masse de catalyseur.

Selon l'invention, la réaction d'estérification a) est conduite en présence d'un excès d'acide carboxylique, et le milieu en pied de distillation est avantageusement soutiré et recyclé par réintroduction en amont ou au cours de la réaction a), de préférence en amont.

Par « en amont » on entend que le milieu en pied de distillation est réintroduit dans le mélange d'acide carboxylique et d'alcool avant qu'il ait réagi.

Dans ce cas, le ratio entre le débit d'alimentation du mélange réactionnel (acide carboxylique + alcool) et le débit du flux de recyclé est avantageusement compris entre 4 et 20, de préférence entre 5 et 15.

Dans ce mode de réalisation, le ratio molaire acide carboxylique/alcool est soit le ratio dans le mélange comprenant l'acide carboxylique et l'alcool avant la réaction lorsque les réactifs sont introduits en mélange, soit le ratio en début de la réaction lorsque les réactifs sont introduits séparément dans la réaction. Ce ratio tient compte de l'apport en acide carboxylique et en alcool provenant du recyclage. Ce ratio molaire acide carboxylique/alcool est compris entre 7 et 25, et plus préférentiellement entre 9 et 20.

Le flux vapeur issu de l'étape b) est avantageusement condensé sous forme liquide en abaissant sa température à une température par exemple comprise entre 15°C et 40°C par passage dans un ou plusieurs condenseurs.

Le flux liquide ainsi obtenu est ensuite envoyé vers un moyen de séparation de phases liquides, de préférence un décanteur séparant la phase organique contenant majoritairement l'ester de l'acide carboxylique et minoritairement l'alcool, de l'eau et des traces d'acide carboxylique et la phase aqueuse contenant majoritairement de l'eau et minoritairement de l'alcool et de l'acide carboxylique.

La phase organique est, de façon avantageuse, réintroduite en partie en tête de distillation afin d'assurer le fonctionnement au reflux de la colonne.

Le taux de reflux est de préférence compris entre 1 et 8 avantageusement entre 2 et 6.

Conformément à l'invention, le restant de la phase organique issu de l'étape d) constitué majoritairement de l'ester de l'acide carboxylique et minoritairement l'alcool, de l'eau et des traces d'acide carboxylique subit ensuite une étape d'extraction liquide/liquide comme précédemment décrit.

Selon le procédé de l'invention, la phase organique comprenant majoritairement l'ester d'acide carboxylique et minoritairement l'alcool, de l'eau et des traces d'acide carboxylique, qui est issue de l'étape d) est appauvrie en alcool et débarrassée de l'acide carboxylique par une opération d'extraction liquide/liquide par mise en contact de ladite phase organique avec une phase aqueuse permettant d'extraire l'alcool et l'acide carboxylique présents dans la phase organique vers la phase aqueuse.

Selon un mode particulier de réalisation de l'invention, la phase aqueuse utilisée pour l'extraction liquide/liquide peut provenir en totalité ou en partie de la phase aqueuse issue de l'étape de séparation des phases d) , cette phase aqueuse ayant de préférence subit une purification par exemple par passage dans un dispositif d'entraînement, notamment une colonne de séparation de l'eau et de l'alcool qu'elle contient.

A la sortie du dispositif d'entraînement, la phase comprenant majoritairement de l'alcool peut avantageusement être recyclée par réintroduction en début de réaction et la phase comprenant majoritairement de l'eau peut être utilisée pour l'extraction liquide/liquide.

Selon un mode préféré de réalisation du procédé de l'invention, la phase organique appauvrie en alcool et débarrassée de l'acide carboxylique récupérée à la sortie de l'étape d'extraction liquide/liquide est soumise à une opération de distillation, par exemple dans une colonne de distillation. Lors de cette étape, on élimine l'eau et l'alcool résiduels qui n'ont pu être éliminés lors de l'étape d'extraction liquide/liquide telle que décrite plus haut dans la présente description et l'on récupère l'ester d'acide carboxylique purifié. L'eau et l'alcool sont éliminés en tête de distillation et l'ester d'acide carboxylique purifié est récupéré en pied de distillation.

Par « purifié » au sens de la présente invention, on entend que l'ester d'acide carboxylique ainsi récupéré comprend moins de 0,1 % en masse d'alcool, de préférence entre 0,015 % et 0,100 % en masse d'alcool, et moins de 0,1 % en masse d'eau, de préférence entre 0,010 % et 0,100 % en masse, et est exempt d'acide carboxylique.

Selon un mode préféré de réalisation, le procédé de l'invention comprend les étapes suivantes :
a) réaction d'estérification entre l'acide carboxylique et l'alcool, de préférence en présence d'un catalyseur,
b) distillation du milieu réactionnel issu de l'étape a), permettant d'obtenir en tête de colonne de distillation un flux vapeur comprenant majoritairement l'ester d'acide carboxylique et minoritairement l'alcool, de l'eau et des traces d'acide carboxylique,
c) condensation du flux vapeur issu de l'étape b),
d) séparation des phases organique et aqueuse, notamment par décantation,
e) extraction liquide/liquide de la phase organique issue de l'étape d),
f) distillation de la phase organique issue de l'étape e),
g) récupération de l'ester d'acide carboxylique purifié.

Il est également décrit ici un dispositif pour la mise en oeuvre du procédé de l'invention.

Ce dispositif, qui se présente le plus souvent sous la forme d'une installation de dimensions industrielles, comprend un dispositif d'extraction liquide/liquide, des moyens d'alimentation du dispositif d'extraction liquide/liquide en phase organique comprenant majoritairement l'ester d'acide carboxylique et minoritairement l'alcool, de l'eau et des traces d'acide carboxylique, et des moyens d'alimentation du dispositif d'extraction liquide/liquide en phase aqueuse, ainsi que par des moyens de récupération des phases aqueuse et organique en sortie du dispositif d'extraction liquide/liquide.

De préférence, ce dispositif comprend en amont également un réacteur, une colonne de distillation dont la partie haute est reliée à des moyens pour traiter le flux vapeur issu de la colonne de distillation, à savoir au moins un condenseur dont la sortie est reliée à un séparateur de phase liquide/liquide, de préférence un décanteur, lui-même relié au dispositif d'extraction liquide/liquide.

### Description des figures

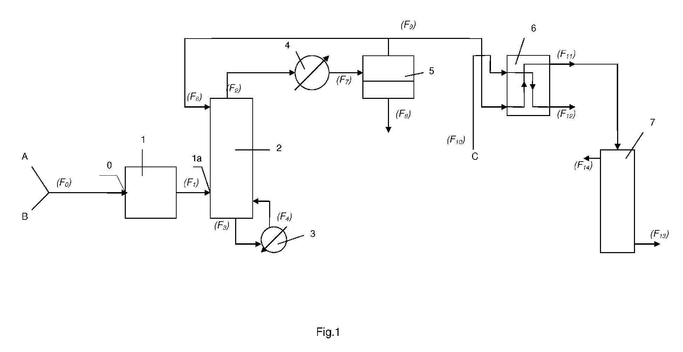

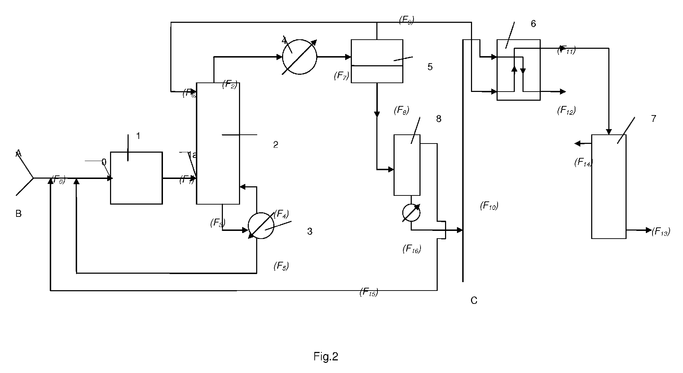

L'invention sera encore explicitée davantage par le biais de la description qui suit, faite en référence aux figures 1 et 2.

La figure 1 est une représentation schématique d'un dispositif préféré pour la mise en oeuvre du procédé selon l'invention comprenant un réacteur 1, une colonne de distillation 2 dont la partie haute est reliée à des moyens pour traiter le flux vapeur issu de la colonne de distillation 2, à savoir au moins un condenseur 4 dont la sortie est reliée à un décanteur 5.

L'alcool A et l'acide carboxylique B forment un flux (*F₀*) qui est introduit en 0 dans un réacteur 1.

Le réacteur est de préférence adiabatique. Il peut être du type parfaitement agité ou du type piston, de préférence du type piston. Le flux (*F₁*) issu de la réaction est introduit en 1 a dans une colonne de distillation 2.

Cette étape vise à obtenir en tête de distillation un flux vapeur (F₂) comprenant majoritairement l'ester de l'acide carboxylique attendu et minoritairement l'alcool, de l'eau et des traces d'acide carboxylique. En pied de distillation, le flux liquide (*F₃*) comprend majoritairement l'acide carboxylique.

L'homme du métier est parfaitement en mesure de choisir les moyens à mettre en oeuvre en fonction de la séparation à effectuer.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et les puretés des produits devant être obtenues en tête et en pied de distillation.

On précisera que la colonne peut être garnie indifféremment de plateaux ou de garnissage ordonné ou tissé, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 40, de préférence de 2 à 20,
- taux de reflux R compris entre 1 et 8, de préférence entre 2 et 6.

Le taux de reflux est défini par le rapport du débit de matière réinjectée de la tête de la colonne vers l'intérieur de la colonne et du débit de phase organique sortant effectivement du décanteur.

Pour effectuer la distillation, l'apport des calories en pied de colonne peut être fait notamment par un échangeur tubes calandre, un échangeur à plaques, un échangeur à serpentin ou par tout autre dispositif équivalent. Le chauffage peut se faire à la vapeur ou par un fluide caloporteur.

Un mode de réalisation préféré consiste à chauffer le mélange en pied de distillation dans un échangeur thermique 3 par prélèvement d'un flux (*F₄*) en pied qui circule en boucle. Plus précisément, le flux (*F₃*) sort en pied de distillation et une fraction (*F₄*) traverse de bas en haut un échangeur thermique et en sortie d'échangeur est introduit sous forme d'un mélange liquide vapeur dans la partie inférieure de la colonne de distillation.

Un autre mode de réalisation consiste à effectuer une circulation forcée du flux (*F₃*) dans l'échangeur à l'aide d'une pompe.

Le flux vapeur (*F₂*), en tête de colonne, comprenant majoritairement l'ester de l'acide carboxylique et minoritairement l'alcool, de l'eau et des traces d'acide carboxylique est condensé de manière à récupérer un flux liquide dont une fraction (*F₆*) est introduite, latéralement en tête de colonne, pour assurer le reflux dans la colonne et l'autre fraction (*F₉*) est traitée dans une étape ultérieure de purification de l'ester de l'acide carboxylique.

En tête de distillation, la récupération du flux comprenant majoritairement l'ester de l'acide carboxylique et minoritairement l'alcool, de l'eau et des traces d'acide carboxylique, à partir du flux (*F₂*) se fait par condensation, par exemple par passage dans un ou plusieurs condenseurs 4.

On refroidit la phase vapeur (*F₂*) et la transforme sous forme liquide par refroidissement en abaissant sa température à une température comprise par exemple entre 15°C et 40°C.

Cette opération est conduite par passage dans un condenseur qui est un appareil classique par exemple un échangeur tubulaire alimenté par un fluide caloporteur (généralement de l'eau) maintenu à une température voisine de la température de refroidissement choisie.

Le nombre et la taille des condenseurs sont choisis en fonction des capacités réfrigérantes des liquides de refroidissement circulant dans les condenseurs.

Dans le cas de condenseurs en série, la phase vapeur en sortie du premier condenseur est introduite dans le deuxième condenseur.

On récupère en sortie du ou des condenseurs, le flux liquide (*F₇*).

Le flux liquide (*F₇*) est introduit dans un décanteur 5 qui sépare la phase aqueuse (*F₈*) de la phase organique (*F₆*+*F₉*).

L'ester attendu présent dans le flux (*F₉*), est envoyé vers un dispositif d'extraction liquide/liquide 6 alimenté, par exemple à contre-courant, par un flux (*F₁₀*) d'eau C.

A la sortie de l'extracteur liquide/liquide 6, on obtient un flux (*F₁₂*) comprenant majoritairement de l'eau et minoritairement l'alcool et des traces d'acide carboxylique, et un flux (*F₁₁*) comprenant majoritairement l'ester d'acide carboxylique, appauvri en alcool, en eau et en acide carboxylique, qui est ultérieurement envoyé vers une colonne de distillation 7 de laquelle on récupère : en tête de distillation un flux (*F₁₄*) comprenant des quantités résiduelles d'alcool et d'eau, et en pied de distillation un flux (*F₁₃*) comprenant l'ester purifié.

La figure 2 est une représentation schématique d'un autre dispositif préféré pour la mise en oeuvre du procédé selon l'invention comprenant un réacteur 1, une colonne de distillation 2 dont la partie haute est reliée à des moyens pour traiter le flux vapeur issu de la colonne de distillation 2, à savoir au moins un condenseur 4 dont la sortie est reliée à un décanteur 5.

L'alcool A et l'acide carboxylique B forment un flux (*F₀*) qui est introduit en 0 dans un réacteur 1.

Le réacteur est de préférence adiabatique. Il peut être du type parfaitement agité ou du type piston, de préférence du type piston. Le flux (*F₁*) issu de la réaction est introduit en 1 a dans une colonne de distillation 2.

Cette étape vise à obtenir en tête de distillation un flux vapeur (*F₂*) comprenant majoritairement l'ester de l'acide carboxylique attendu et minoritairement l'alcool, de l'eau et des traces d'acide carboxylique. En pied de distillation, le flux liquide (*F₃*) comprend majoritairement l'acide carboxylique.

La colonne de distillation est avantageusement, mais non limitativement, une colonne ayant les spécifications suivantes :
- nombre d'étages théoriques : de 1 à 40, de préférence de 2 à 20,
- taux de reflux R compris entre 1 et 8, de préférence entre 2 et 6.

Un mode de réalisation préféré consiste à chauffer le mélange en pied de distillation dans un échangeur thermique 3 par prélèvement d'un flux (*F₄*) en pied qui circule en boucle. Plus précisément, le flux (*F₃*) sort en pied de distillation et une fraction (*F₄*) traverse de bas en haut un échangeur thermique et en sortie d'échangeur est introduit sous forme d'un mélange liquide vapeur dans la partie inférieure de la colonne de distillation.

Un autre mode de réalisation consiste à effectuer une circulation forcée du flux (*F₃*) dans l'échangeur à l'aide d'une pompe.

De façon avantageuse, le flux (*F₅*) en sortie d'échangeur est réacheminé en amont du réacteur 1 par exemple par l'intermédiaire de pompes. Si le flux (*F₅*) est réacheminé en amont du réacteur 1, alors le ratio du débit du flux (*F₅*) sur le débit du flux (*F₀*) est de préférence compris entre 4 et 20.

Le flux vapeur (*F₂*), en tête de colonne, comprenant majoritairement l'ester de l'acide carboxylique et minoritairement l'alcool, de l'eau et des traces d'acide carboxylique est condensé de manière à récupérer un flux liquide dont une fraction (*F₆*) est introduite, latéralement en tête de colonne, pour assurer le reflux dans la colonne et l'autre fraction (*F₉*) est traitée dans une étape ultérieure de purification de l'ester de l'acide carboxylique.

En tête de distillation, la récupération du flux comprenant majoritairement l'ester de l'acide carboxylique et minoritairement l'alcool, de l'eau et des traces d'acide carboxylique à partir du flux (*F₂*) se fait par condensation, par exemple par passage dans un ou plusieurs condenseurs 4.

On refroidit la phase vapeur (*F₂*) et la transforme sous forme liquide par refroidissement en abaissant sa température à une température comprise par exemple entre 15°C et 40°C.

Cette opération est conduite comme décrite pour la figure 1.

On récupère en sortie du ou des condenseurs, le flux liquide (*F₇*).

Le flux liquide (*F₇*) est introduit dans un décanteur 5 qui sépare la phase aqueuse (*F₈*) de la phase organique (*F₆*+*F₉*).

La phase (*F₈*) peut être retraitée par passage dans un dispositif d'entraînement 8 permettant de récupérer d'une part une phase comprenant majoritairement de l'eau (*F₁₆*) et d'autre part une phase comprenant majoritairement de l'alcool et de l'ester de l'acide carboxylique en mélange (*F₁₅*). Le flux (*F₁₅*) peut être réacheminé en amont du réacteur 1 par exemple par l'intermédiaire de pompes.

L'ester attendu présent dans le flux (*F₉*) est envoyé vers un dispositif d'extraction liquide/liquide 6 alimenté, par exemple à contre-courant, par un flux (*F₁₀*) d'eau C, qui peut provenir en totalité ou pour partie du flux (*F₁₆*).

A la sortie de l'extracteur liquide/liquide 6, on obtient un flux (*F₁₂*) comprenant majoritairement l'eau et minoritairement l'alcool et des traces d'acide carboxylique et un flux (*F₁₁*) comprenant majoritairement l'ester d'acide carboxylique, appauvri en alcool, en eau et en acide carboxylique, qui est ultérieurement envoyé vers une colonne de distillation 7 de laquelle on récupère : en tête de distillation un flux (*F₁₄*) comprenant des quantités résiduelles d'alcool et d'eau, et en pied de distillation un flux (*F₁₃*) comprenant l'ester purifié.

### Avantages

Le procédé de l'invention est particulièrement intéressant en raison des avantages qu'il procure.

Un des avantages du procédé de la présente invention est que l'on consomme moins d'énergie.

En effet, la présence de l'étape d'extraction liquide/liquide rend la deuxième distillation de la phase organique issue de l'étape extraction liquide/liquide, beaucoup plus économique du point de vue énergétique.

les exemples qui suivent, illustrent l'invention sans toutefois la limiter.

### Exemples

Pour une meilleure compréhension, les exemples ci-après sont décrits en référence à la figure 2. Les exemples suivants sont donnés pour une production finale de 100 kg/h d'acétate d'éthyle.

### Exemple 1 selon l'invention (E-1) :

On alimente un réacteur d'estérification 1 par introduction simultanée d'éthanol A avec un débit de 56 kg/h et d'acide acétique B avec un débit de 69 kg/h. Le débit total d'alimentation est appelé débit (*F₀*).

On introduit également dans le réacteur d'estérification 1, 2,3 kg d'acide méthanesulfonique comme catalyseur. Le réacteur d'estérification 1 a une capacité de 230 kg.

De l'acide méthanesulfonique est régulièrement injecté et purgé de manière à maintenir l'activité catalytique.

La température d'entrée du réacteur est de 117°C. Le réacteur est adiabatique.

Le flux de sortie du réacteur d'estérification (*F₁*) alimente ensuite une colonne d'estérification 2. Cette colonne comprend 16 plateaux théoriques. Elle fonctionne à une pression de 1,5 bars absolus et à une température en pied de colonne de 118°C.

Le flux provenant du pied de la colonne d'estérification (*F₅*) et contenant majoritairement de l'acide acétique est recyclé vers l'entrée du réacteur d'estérification 1.

Le recyclage dudit flux (*F₅*) est effectué de telle manière que le ratio molaire acide acétique sur éthanol soit de 16 à l'entrée 0 du réacteur 1. Dans ces conditions, le ratio massique entre le débit de recyclage (*F₅*) et le débit d'alimentation (*F₀*) est de 12.

Le flux en tête de colonne (*F₂*) contenant majoritairement l'acétate d'éthyle (90 % en masse), l'eau de réaction (8 % en masse) et 2,0 % en en masse d'éthanol non converti est condensé dans un échangeur 4 puis envoyé dans un décanteur 5 dans lequel deux phases sont séparées.

La phase aqueuse (*F₈*) contient majoritairement de l'eau et minoritairement de l'éthanol et de l'acétate d'éthyle. Cette phase aqueuse (*F₈*) est envoyée vers une colonne de distillation en vue de séparer l'eau (*F₁₆*) et un flux comprenant majoritairement l'éthanol non converti et l'acétate d'éthyle qui est recyclé au réacteur d'estérification (*F₁₅*).

La phase organique (*F₆*+*F₉*) en sortie de décanteur 5 contient majoritairement de l'acétate d'éthyle et, à saturation, de l'eau, de l'éthanol et des traces d'acide acétique.

Une partie (*F₆*) de cette phase organique est renvoyée vers la tête de colonne pour assurer le reflux avec un taux de reflux *(F₆)l(F₉)* de 3,6.

L'autre partie (*F₉*) de la phase organique est ensuite envoyée vers un extracteur liquide/liquide 6 fonctionnant à contre-courant à une température comprise de 30°C, et à pression atmosphérique.

Le ratio entre les débits des phases organique et aqueuse au début de l'étape d'extraction liquide/liquide est de 6. Le flux (*F₁₀*) d'eau C, provient du flux (*F₁₆*).

A la sortie de l'extracteur liquide/liquide 6, on obtient un flux (*F₁₂*) comprenant majoritairement l'eau et un flux (*F₁₁*) comprenant majoritairement l'ester qui est ultérieurement envoyé vers une colonne de distillation 7 de manière à éliminer l'eau et l'éthanol qu'il contient pour obtenir la qualité désirée du produit final (*F₁₃*).

La teneur en éthanol dans l'acétate d'éthyle après cette distillation finale est inférieure à 0,03 % en poids.

Exemple 1 comparatif - Procédé sans extraction liquide/liquide (EC-1) :

On reproduit le procédé selon l'exemple 1 mis à part l'extraction liquide/liquide. La partie (*F₉*) de la phase organique est ensuite directement distillée de manière à éliminer l'eau et l'éthanol qu'il contient pour obtenir la qualité désirée du produit final.

### Exemple 2 (E-2) :

On reproduit le procédé ci-dessus décrit dans l'exemple 1 pour un ratio molaire acide acétique/alcool de 7 à l'entrée 0 du réacteur 1.

### Exemple 2 comparatif (EC-2) :

On reproduit le procédé ci-dessus décrit dans l'exemple 1 comparatif pour un ratio molaire acide acétique/alcool de 7 à l'entrée 0 du réacteur 1.

### Exemple 3 (E-3) :

On reproduit le procédé ci-dessus décrit dans l'exemple 1 pour un ratio molaire acide acétique/alcool de 25 à l'entrée 0 du réacteur 1.

### Exemple 3 comparatif (EC-3) :

On reproduit le procédé ci-dessus décrit dans l'exemple 1 comparatif pour un ratio molaire acide acétique/alcool de 25 à l'entrée 0 du réacteur 1.

### Exemple 4 (E-4) :

On reproduit le procédé ci-dessus décrit dans l'exemple 1 pour un ratio molaire acide acétique/alcool de 5 à l'entrée 0 du réacteur 1.

### Exemple 4 comparatif (EC-4) :

On reproduit le procédé ci-dessus décrit dans l'exemple 1 comparatif pour un ratio molaire acide acétique/alcool de 5 à l'entrée 0 du réacteur 1.

### Résultats :

Les résultats sont présentés dans le tableau ci-dessous dans lequel la comparaison entre les exemples s'entend tous les autres paramètres étant égaux par ailleurs. On a mesuré la consommation de vapeur globale de l'ensemble de l'installation pour chacun des exemples.

| Paramètres | Unité | E-4 | EC-4 | E-2 | EC-2 | E-1 | EC-1 | E-3 | EC-3 |
|---|---|---|---|---|---|---|---|---|---|
| Ratio acide acétique / éthanol | mol/mol | 5 | 5 | 7 | 7 | 16 | 16 | 25 | 25 |
| *(F₅)*/*(F₀)* | kg/kg | 3 | 2,8 | 4,4 | 4,4 | 12 | 12 | 20 | 20 |
| TT éthanol | % | 86,1 | 87,5 | 86,6 | 86,9 | 83,3 | 83,7 | 75,1 | 76,1 |
| Ethanol en tête colonne (*F₂*) | % masse | 3,2 | 3,4 | 2,6 | 2,9 | 1,8 | 2,0 | 1,8 | 1,8 |
| Acide acétique dans le flux (*F₉*) | ppm | 49 | 50 | 48 | 50 | 50 | 49 | 48 | 50 |
| Acide acétique dans le flux (*F₁₁*) | ppm | 31 | - | 29 | - | 28 | - | 29 | - |
| Ethanol après extraction | % masse | 2,0 | - | 1,6 | - | 1,1 | - | 1,1 | - |
| Ethanol après distillation finale | % masse | 0,03 | 0,6 | 0,03 | 0,03 | 0,03 | 0,03 | 0,03 | 0,03 |
| Consommation de vapeur globale | MWh/t | 1,6 | 2,5 | 1,4 | 2,3 | 1,3 | 1,5 | 1,3 | 1,5 |

On constate que les procédés des exemples selon l'invention permettent de diminuer la consommation de vapeur globale de l'installation (mesurée sur l'ensemble des dispositifs de l'installation), comparés à des procédés sans extraction liquide/liquide, toutes les caractéristiques étant égales par ailleurs.

On constate également que l'augmentation du ratio molaire acide acétique / éthanol à l'entrée du réacteur d'estérification permet de diminuer la consommation énergétique de la colonne de distillation finale de l'acétate d'éthyle et donc de la consommation globale de l'unité.

## Revendications

1. Procédé d'obtention d'un ester d'acide carboxylique à partir d'une phase organique issue d'une réaction d'estérification entre un acide carboxylique et un alcool, ladite phase organique comprenant au moins majoritairement l'ester d'acide carboxylique et minoritairement l'alcool, de l'eau et des traces d'acide carboxylique,
**caractérisé par le fait qu'**il comprend une étape de séparation au cours de laquelle la phase organique est appauvrie en alcool et débarrassée de l'acide carboxylique par une opération d'extraction liquide/liquide par mise en contact de ladite phase organique avec une phase aqueuse permettant d'extraire l'alcool et l'acide carboxylique présents dans la phase organique vers la phase aqueuse
et **par le fait que** la phase organique comprenant au moins majoritairement l'ester d'acide carboxylique et minoritairement l'alcool, de l'eau et des traces d'acide carboxylique est issue des étapes suivantes :
a) réaction d'estérification entre l'acide carboxylique et l'alcool en présence d'un catalyseur homogène ou hétérogène,
b) distillation du milieu réactionnel issu de l'étape a), permettant d'obtenir en tête de colonne de distillation un flux vapeur comprenant majoritairement l'ester d'acide carboxylique et minoritairement de l'alcool, de l'eau et des traces d'acide carboxylique,
c) condensation du flux vapeur issu de l'étape b)
d) séparation des phases organique et aqueuse, notamment par décantation
et **par le fait que** l'alcool et l'acide carboxylique sont introduits à l'étape a) avec un ratio molaire acide carboxylique/alcool compris entre 7 et 25 ; l'acide carboxylique étant l'acide acétique et l'alcool étant choisi parmi l'éthanol, le butanol, le n-propanol et le cyclohexanol,
et **par le fait que** le ratio entre les débits des phases organique et aqueuse au début de l'étape d'extraction liquide/liquide est compris entre 4 et 8.

2. Procédé selon la revendication 1 **caractérisé par le fait qu'**après l'extraction liquide/liquide, on obtient d'une part, une phase organique enrichie en ester d'acide carboxylique, appauvrie en alcool et débarrassée de l'acide carboxylique, et d'autre part, une phase aqueuse comprenant majoritairement de l'eau, de l'alcool et des traces d'acide carboxylique.

3. Procédé selon l'une des revendications 1 ou 2 **caractérisé par le fait que** l'opération d'extraction liquide/liquide est conduite à une température comprise entre 15 et 50°C, et est mise en oeuvre à une pression comprise entre 0,5 et 5 bars absolus.

4. Procédé selon l'une des revendications 1 à 3, **caractérisé par le fait que** l'alcool et l'acide carboxylique sont introduits avec un ratio molaire acide carboxylique/alcool compris entre 9 et 20.

5. Procédé selon l'une des revendications 1 à 4, **caractérisé par le fait que** la phase aqueuse permettant d'extraire l'alcool et l'acide carboxylique présents dans la phase organique vers la phase aqueuse est de l'eau ou provient en totalité ou en partie de la phase aqueuse issue de l'étape d) ayant subit une purification.

6. Procédé selon l'une des revendications 1 à 5, **caractérisé par le fait que** l'alcool est l'éthanol.

7. Procédé selon l'une des revendications 1 à 6, **caractérisé par le fait que** la phase organique appauvrie en alcool et débarrassée de l'acide carboxylique récupérée à la sortie de l'étape d'extraction liquide/liquide est soumise à une opération de distillation permettant de récupérer en tête de distillation l'eau et l'alcool qui n'ont pu être éliminés lors de l'étape d'extraction liquide/liquide, et en pied de distillation l'ester d'acide carboxylique purifié.

## Patentansprüche

1. Verfahren zum Erhalt eines Carbonsäureesters aus einer aus einer Veresterungsreaktion zwischen einer Carbonsäure und einem Alkohol resultierenden organischen Phase, die mindestens hauptsächlich den Carbonsäureester und nebensächlich den Alkohol, Wasser und Carbonsäurespuren umfasst, **dadurch gekennzeichnet, dass** es einen Trennschritt umfasst, in dessen Verlauf die organische Phase durch einen Flüssig/Flüssig-Extraktionsarbeitsgang durch Inberührungbringen der organischen Phase mit einer wässrigen Phase, die es ermöglicht, den in der organischen Phase vorliegenden Alkohol und die in der organischen Phase vorliegende Carbonsäure in die wässrige Phase zu extrahieren, an Alkohol verarmt und von der Carbonsäure befreit wird,
und dadurch, dass die organische Phase, die mindestens hauptsächlich den Carbonsäureester und nebensächlich den Alkohol, Wasser und Carbonsäurespuren umfasst, aus den folgenden Schritten resultiert:
a) Veresterungsreaktion zwischen der Carbonsäure und dem Alkohol in Gegenwart eines homogenen oder heterogenen Katalysators,
b) Destillation des Reaktionsmediums aus Schritt a), wodurch am Kopf der Destillationssäule ein Dampfstrom, der hauptsächlich den Carbonsäureester und nebensächlich Alkohol, Wasser und Carbonsäurespuren umfasst, erhalten werden kann,
c) Kondensation des Dampfstroms aus Schritt b),
d) Trennung der organischen Phase und der wässrigen Phase, insbesondere durch Absetzen,
und dadurch, dass der Alkohol und die Carbonsäure in einem Molverhältnis von Carbonsäure zu Alkohol zwischen 7 und 25 in Schritt a) eingetragen werden; wobei es sich bei der Carbonsäure um Essigsäure handelt und der Alkohol aus Ethanol, Butanol, n-Propanol und Cyclohexanol ausgewählt wird,
und dadurch, dass das Verhältnis zwischen den Strömungsraten der organischen Phase und der wässrigen Phase zu Beginn des Flüssig/Flüssig-Extraktionsschritts zwischen 4 und 8 liegt.

2. Verfahren nach Anspruch 1, **dadurch gekennzeichnet, dass** man nach der Flüssig/Flüssig-Extraktion einerseits eine an Carbonsäureester angereicherte, an Alkohol verarmte und von der Carbonsäure befreite organische Phase und andererseits eine wässrige Phase, die hauptsächlich Wasser, Alkohol und Carbonsäurespuren umfasst, erhält.

3. Verfahren nach einem der Ansprüche 1 oder 2, **dadurch gekennzeichnet, dass** der Flüssig/Flüssig-Extraktionsarbeitsgang bei einer Temperatur zwischen 15 und 50°C und einem Druck zwischen 0,5 und 5 bar absolut durchgeführt wird.

4. Verfahren nach einem der Ansprüche 1 bis 3, **dadurch gekennzeichnet, dass** der Alkohol und die Carbonsäure in einem Molverhältnis von Carbonsäure zu Alkohol zwischen 9 und 20 eingetragen werden.

5. Verfahren nach einem der Ansprüche 1 bis 4, **dadurch gekennzeichnet, dass** es sich bei der wässrigen Phase, die es ermöglicht, den in der organischen Phase vorliegenden Alkohol und die in der organischen Phase vorliegende Carbonsäure in die wässrige Phase zu extrahieren, um Wasser handelt oder ganz oder teilweise aus der einer Reinigung unterworfenen wässrigen Phase aus Schritt d) stammt.

6. Verfahren nach einem der Ansprüche 1 bis 5, **dadurch gekennzeichnet, dass** es sich bei dem Alkohol um Ethanol handelt.

7. Verfahren nach einem der Ansprüche 1 bis 6, **dadurch gekennzeichnet, dass** die an Alkohol verarmte und von Carbonsäure befreite organische Phase, die am Ausgang des Flüssig/Flüssig-Extraktionsschritts gewonnen wird, einem Destillationsarbeitsgang unterworfen wird, der es ermöglicht, am Kopf der Destillation das Wasser und den Alkohol, die während des Flüssig/Flüssig-Extraktionsschritts nicht entfernt werden konnten, und am Sumpf der Destillation der gereinigten Carbonsäureester zu gewinnen.

## Claims

1. Process for producing a carboxylic acid ester from an organic phase resulting from an esterification reaction between a carboxylic acid and an alcohol, said organic phase comprising at least predominantly the carboxylic acid ester and to a minor extent the alcohol, water and traces of carboxylic acid, **characterized in that** it comprises a separation stage during which the organic phase is depleted in alcohol and freed from the carboxylic acid by a liquid/liquid extraction operation by bringing said organic phase into contact with an aqueous phase which makes it possible to extract the alcohol and the carboxylic acid present in the organic phase toward the aqueous phase, and **in that** the organic phase comprising at least predominantly the carboxylic acid ester and to a minor extent the alcohol, water and traces of carboxylic acid results from the following stages:
a) esterification reaction between the carboxylic acid and the alcohol, in the presence of a homogeneous or heterogeneous catalyst,
b) distillation of the reaction medium resulting from stage a), making it possible to obtain, at the distillation column top, a vapor stream comprising predominantly the carboxylic acid ester and to a minor extent alcohol, water and traces of carboxylic acid,
c) condensation of the vapor stream resulting from stage b),
d) separation of the organic and aqueous phases, in particular by settling, **in that** the alcohol and the carboxylic acid are introduced and in stage a) with a carboxylic acid/alcohol molar ratio of between 7 and 25, the carboxylic acid being acetic acid and the alcohol being chosen from ethanol, butanol, n-propanol and cyclohexanol, and **in that** the ratio of the flow rate of the organic phase to the flow rate of the aqueous phase at the start of the liquid/liquid extraction stage is between 4 and 8.

2. Process according to Claim 1, **characterized in that**, after the liquid/liquid extraction, on the one hand, an organic phase enriched in carboxylic acid ester, depleted in alcohol and freed from the carboxylic acid is obtained and, on the other hand, an aqueous phase comprising predominantly water, alcohol and traces of carboxylic acid is obtained.

3. Process according to either of Claims 1 and 2, **characterized in that** the liquid/liquid extraction operation is carried out at a temperature of between 15 and 50°C, and is carried out at a pressure of between 0.5 and 5 bar absolute.

4. Process according to one of Claims 1 to 3, **characterized in that** the alcohol and the carboxylic acid are introduced with a carboxylic acid/alcohol molar ratio of between 9 and 20.

5. Process according to one of Claims 1 to 4, **characterized in that** the aqueous phase which makes it possible to extract the alcohol and the carboxylic acid present in the organic phase toward the aqueous phase is water or originates, in all or in part, from the aqueous phase resulting from stage d) which has been subjected to a purification.

6. Process according to one of Claims 1 to 5, **characterized in that** the alcohol is ethanol.

7. Process according to one of Claims 1 to 6, **characterized in that** the organic phase depleted in alcohol and freed from the carboxylic acid recovered at the outlet of the liquid/liquid extraction stage is subjected to a distillation operation which makes it possible to recover, at the distillation top, the water and the alcohol which could not be removed during the liquid/liquid extraction stage and, at the distillation bottom, the purified carboxylic acid ester.
